# EUROPEAN PATENT SPECIFICATION

(11) **EP 4 327 734 B1**
(45) Date of publication and mention of the grant of the patent: **30.09.2026**
(21) Application number: 22191644.8
(22) Date of filing: 23.08.2022
(51) Int. Cl.: A61B 5/022

(54) **TOURNIQUET**
ABBINDUNGSSYSTEM
TOURNIQUET

(43) Date of publication of application: 28.02.2024
(73) Proprietor: Contemporary Han Cloud Co., Ltd, Taipei City 10684 (TW)
(72) Inventor: Kuo, Yu-Cheng, Taipei City, Taiwan 10684 (TW)
(74) Representative: Straus, Alexander

(56) References cited:
- EP-A1- 1 688 089
- DE-T5- 112013 002 059
- US-A1- 2008 177 187

## Description

### BACKGROUND OF THE INVENTION

### 1. Field of the Invention

The present invention relates to a tourniquet, in particular to a tourniquet comprising a measuring portion and an elastic band portion connected in series with each other to form a radial resonant ring corresponding to the radial fluctuation of the pulse.

### 2. The Prior Art

When a pulse diagnosis device or an electronic blood pressure monitor is used for measuring a blood pressure wave of an organism (e.g., a human or another animal) or for performing a pulse diagnosis, a tourniquet is touched to a position of a pulse for performing the measurement to understand a physiological condition of the organism. However, accuracy of the measurement is affected by the tourniquet, especially when it is needed to measure harmonics of the blood pressure wave with high accuracy. Quality of the tourniquet has a major impact. Thus, design of a proper tourniquet is an important problem to be solved.

Prior art devices are known from US2008/177187, EP1688089 and DE112013002059.

### SUMMARY OF THE INVENTION

An objective of the present invention is to provide a tourniquet, comprising: a covering bag, including a surrounding device, a measuring portion, and an elastic band portion connected in series, wherein the measuring portion defines an accommodating space and is made of a non-elastic material, and the elastic band portion is made of an elastic material; and an air bag device, having an elastic air bag, wherein the air bag device is put into the accommodating space such that the elastic air bag and the measuring portion overlap.

According to the present invention, the surrounding device includes a connecting member, so that the tourniquet forms a ring structure to locate the measuring portion at a pulse position to be measured.

According to the present invention, the ring structure has a radial elasticity coefficient, and the radial elasticity coefficient is determined according to a frequency of a harmonic of a blood pressure wave to be measured.

According to an embodiment of the present invention, the covering bag further includes a fixing structure, the air bag device includes a fixing element, and the fixing element is coupled with the fixing structure to fix the covering bag and the air bag device.

According to an embodiment of the present invention, a surface of the accommodating space of the measuring portion has a non-slip material part, and the non-slip material part is in contact with the fixing element to prevent displacement of the air bag device.

According to an embodiment of the present invention, the fixing structure of the covering bag has a plurality of holes and a plurality of fixing holes.

According to an embodiment of the present invention, the fixing element of the air bag device has an air inlet pipe, an air outlet pipe, and a plurality of fixation pillars, and the air inlet pipe and the air outlet pipe communicate with the elastic air bag.

According to an embodiment of the present invention, after the air bag device is put into the accommodating space, the air inlet pipe, the air outlet pipe, and the plurality of fixation pillars respectively pass through corresponding holes and fixing holes, so that the tourniquet is coupled to the measurement device by the plurality of fixation pillars.

According to an embodiment of the present invention, the measuring portion and the elastic band portion are connected in series by an elastomeric knitting technique.

According to an embodiment of the present invention, the elastomeric knitting technique is a jump knitting method, or performs stitching with an elastic thread.

In the present disclosure, the radial resonant ring formed by connecting the measuring portion and the elastic band portion of the tourniquet in series can reduce the influence of non-radial vibration, so as to improve the sensitivity and accuracy of the radial resonance.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.
FIG. 1 is a schematic diagram of a tourniquet according to an embodiment of the present invention.
FIG. 2 is a schematic diagram of a covering bag according to an embodiment of the present invention.
FIG. 3 is a schematic diagram of an air bag device and a fixing element therein according to an embodiment of the present invention.
FIG. 4 is a schematic diagram illustrating a ring structure formed by the tourniquet according to an embodiment of the present invention.
FIG. 5 is a schematic diagram of a fixing structure of the tourniquet according to an embodiment of the present invention.

### DETAILED DESCRIPTION OF THE PREFERRED EMBODIMENT

In the following detailed description of the embodiments of the present invention, reference is made to the accompanying drawings, which are shown to illustrate the specific embodiments in which the present disclosure may be practiced. These embodiments are provided to enable those skilled in the art to practice the present disclosure. It is understood that other embodiments may be used and that changes can be made to the embodiments without departing from the scope of the present invention. The following description is therefore not to be considered as limiting the scope of the present invention.

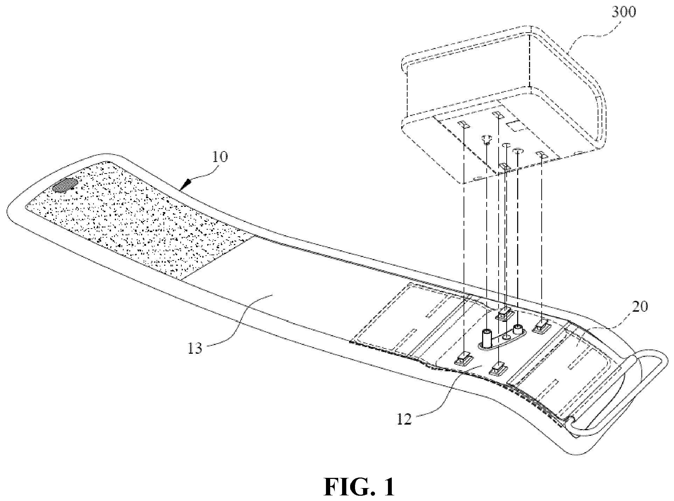

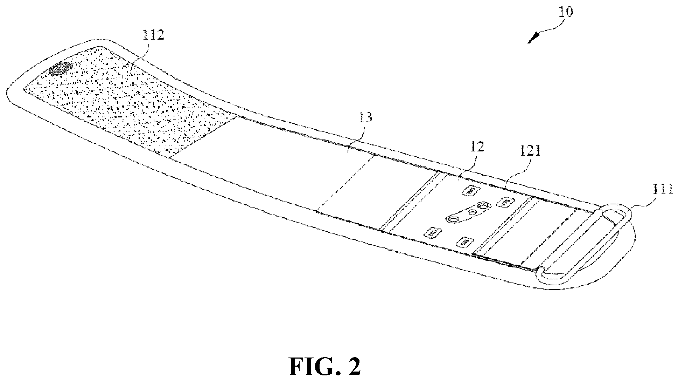

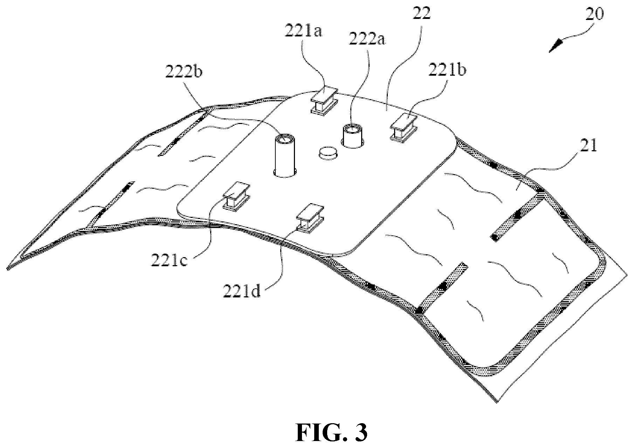

The tourniquet of the present invention is used in a blood pressure wave measurement device to apply a pressure to a pulse of an organism, so as to assist the blood pressure wave measurement device to sense the blood pressure wave of the organism. The blood pressure wave measurement device can be a pulse diagnostic instrument, an electronic blood pressure monitor, etc. The organism can be a human or other animals that the tourniquet can be used to assist in measuring blood pressure waves, such as dogs, cats, etc. Referring to FIGs. 1-3, which are schematic diagrams of the tourniquet according to an embodiment of the present invention, wherein the tourniquet comprises a covering bag 10 and an air bag device 20, wherein the covering bag 10 includes a surrounding device, a measuring portion 12, and an elastic band portion 13 connected in series, and the measuring portion 12 can define an accomodating space 121. The measuring portion 12 is the part where the tourniquet is connected to a measurement device 300, so that the tourniquet can assist the measurement device 300 to measure the blood pressure wave. Referring to FIG. 3, the air bag device 20 has an elastic air bag 21 and may include a fixing element 22 corresponding to the measuring portion 12, wherein the air bag device 20 can be put into the accomodating space 121, so that the elastic air bag 121 overlaps with the measuring portion 12 and can also extend to the elastic band portion 13.

The surrounding device of the covering bag 10 may include a connecting member 111 for making the two ends of the covering bag 10 surround each other, so that the tourniquet of the present invention forms a ring structure as a whole, to be located at the pulse position of the organism to be measured. Therefore, when the pulse position to be measured is the organism's wrist, arm, finger, or neck, the tourniquet of the present invention can surround it to exert pressure on the artery at the position. The ring structure formed by the tourniquet of the present invention can form a radial resonant ring at the pulse position to be measured, so as to fully undulate with the relaxation and contraction of the pulse, so that the measurement device 300 can measure the blood pressure wave more accurately.

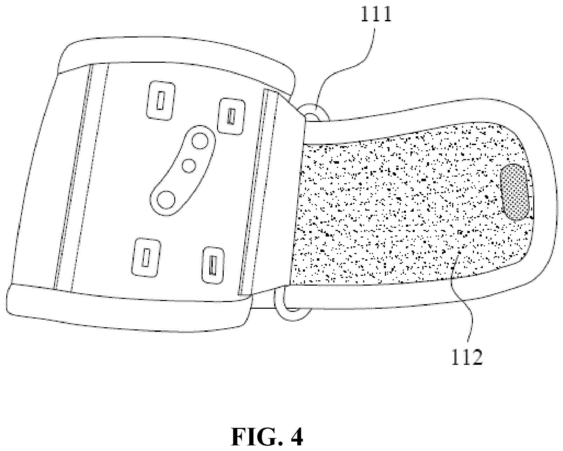

In a preferred embodiment of the present invention, referring to FIG. 4, the connecting member 111 can be a buckle made of metal material, and can be used with a Hook and loop fastener 112 to fix the formed ring structure. More specifically, the buckle is disposed at one end of the covering bag 10, and after the other end of the covering bag 10 is passed through the buckle, the tourniquet of the present invention forms the aforementioned ring structure, and is matched with the Hook and loop fastener 112 disposed on the other end of the covering bag 10 to fix the ring structure. For example, the hook surface and the rough surface of the Hook and loop fastener 112 can be disposed on the same side of the covering bag 10, and the hook surface and the rough surface of the Hook and loop fastener 112 can be adhered by folding the covering bag 10 backwards to fix the ring structure of the tourniquet of the present invention.

In the tourniquet of the present invention, the measuring portion 12 is placed at the position corresponding to the radial artery of the organism or the position corresponding to the artery which can be encircled by the tourniquet (such as a finger or an arm), and is made of a non-elastic material (or inelastic material), such as inelastic leather, inelastic man-made fiber, etc. The elastic band portion 13 can be connected in series with the measuring portion 12, and its constituent material is elastic. The elastic band portion 13 and the measuring portion 12 are respectively designed as elastic and inelastic, the purpose of which is to make the ring structure formed by the two have a radial elasticity coefficient that can generate resonance with the blood pressure wave, so as to form a radial resonant ring. Specifically, the measuring portion 12 is in contact with the artery to be measured to obtain the blood pressure wave (i.e., the radial fluctuation of the artery), and since it is made of a non-elastic material, the measuring portion 12 itself would not generate radial and non-radial resonances (or only tiny resonances) due to the vibration of blood pressure waves. Therefore, after being connected in series with the elastic band portion 13, the mainly radial resonance can be generated along with the expansion and contraction of the elastic band portion 13, and the resonance in other directions would be greatly reduced. Therefore, the radial resonant ring formed by connecting the measuring portion 12 and the elastic band portion 13 in series can reduce the influence of non-radial vibration, so as to improve the sensitivity and accuracy of the radial resonance, so that the vibration of the blood pressure wave can be more efficiently transmitted to the measurement device 300 to improve the measurement accuracy.

In an embodiment of the present invention, the radial elasticity coefficient of the ring structure formed by the tourniquet can be determined or selected according to a frequency of a harmonic of the blood pressure wave to be measured, so that the ring structure can resonate with the harmonic to be measured and thus the accuracy of the measurement can be improved. After the radial elasticity coefficient is determined, the tourniquet can be made in any feasible way to make the ring structure have the determined radial elasticity coefficient. For example, the material with proper elasticity, such as elastic cotton fabric, can be used to make the elastic band portion 13; the width of the elastic band portion 13 can be changed to adjust the radial elasticity coefficient of the ring structure. Since the blood pressure wave can be regarded as a periodic wave, the Fourier transform can be performed on the blood pressure wave obtained by the measurement device 300 to generate harmonics of the blood pressure wave. The importance of the harmonics of the blood pressure wave lies in that they are corresponding to meridians of a human body described in the theory of Chinese medicine. For instance, Table 1 shows the meridians corresponding to harmonics of frequencies of n times the fundamental frequency of the blood pressure wave of the human body (i.e. n-th harmonic, n is an integer from 1 to 10):

**Table 1**

| n-th harmonic | |
|---|---|
| n=1 | Jueyin Liver Channel of Foot |
| n=2 | Shaoyin Kidney Channel of Foot |
| n=3 | Taiyin Spleen Channel of Foot |
| n=4 | Taiyin Lung Channel of Hand |
| n=5 | Yangming Stomach Channel of Foot |
| n=6 | Shaoyang Gallbladder Channel of Foot |
| n=7 | Taiyang Bladder Channel of Foot |
| n=8 | Yangming Large Intestine Channel of Hand |
| n=9 | Shaoyang Sanjiao Channel of Hand |
| n=10 | Taiyang Small Intestine Channel of Hand |

Each harmonic can display an energy state of the corresponding meridian, which has physiological and pathological significance. Thus, it can greatly help the analysis and diagnosis of Chinese medicine by measuring these harmonics of the blood pressure wave of the human body. In the above embodiment, the tourniquet can assist the measurement device 300 to perform more accurate measurement, so as to obtain accurate information of specific harmonics of the blood pressure wave. To achieve this goal, the tourniquet should have specific physical conditions to resonate with the specific harmonic. Specifically, the tourniquet may oscillate with beats of the pulse when it is used for measuring the blood pressure wave. Thus, the ring structure formed by the tourniquet should have an appropriate radial elasticity coefficient to oscillate as a radial resonant ring to fully match relaxation and contraction of the pulse. If the radial elasticity coefficient is too small or large, the tourniquet may be too soft or hard, which results that no data is generated or damping is too high. As a result, an inaccurate measurement result is obtained. Furthermore, a harmonic of a higher frequency needs a larger radial elasticity coefficient to achieve a resonance, so as to obtain accurate harmonic information. Conversely, a harmonic of a lower frequency needs a smaller radial elasticity coefficient to achieve the resonance. In other words, the radial elasticity coefficient of the radial resonant ring has a specific correspondence with the frequency of the harmonic to be measured. Thus, it can improve the resonance of the radial resonant ring with the harmonic to be measured and thus obtain accurate information of the harmonic to be measured, by selecting or determining the radial elasticity coefficient of the radial resonant ring according to the frequency of the harmonic to be measured.

Preferably, the radial elasticity coefficient of the radial resonant ring is corresponding to a pressure-strain modulus Ep. In hemodynamics, the pressure-strain modulus Ep is used for representing an elasticity coefficient of a blood vessel, which is defined as Ep = ΔP*R₀/ΔR₀, where Ep is in a unit of dyn/cm², R₀ is a radius of the blood vessel, and ΔR₀ is a length difference compared with R₀, and ΔP is the amount of change of a pressure. The above equation can be rewritten as Ep = ΔP/(ΔR₀/R₀), and ΔR₀/R₀ is the length difference per unit radial length. Thus, Ep can be regarded as a radial elasticity coefficient of the blood vessel. Furthermore, ΔP is a pressure applied by harmonics and ΔR₀ is a change of a radial length caused by the pressure, if the blood pressure wave is decomposed into the harmonics. In other words, each harmonic has its corresponding pressure-strain modulus Ep. Therefore, a better resonance with the blood vessel can be obtained and a more accurate measurement result can be obtained, if the radial elasticity coefficient of the radial resonant ring can be matched to the pressure-strain modulus Ep of each harmonic. For example, Table 2 shows the correspondence between n-th harmonics and the pressure-strain modulus Ep (in a unit of dyn/cm²). It should be noted that Table 2 is only for exemplifying the present invention, and the scope of the present invention is not limited hereto.

**Table 2**

| n-th harmonic | Pressure-strain modulus Ep |
|---|---|
| 1-4 | 4.22*10⁶-5.77*10⁶ |
| 5-9 | 5.77*10⁶-9.82*10⁶ |
| ≥10 | >9.86*10⁶ |

In one embodiment of the present invention, the elastic band portion 13 includes a plurality of elastic bands for adjusting or switching the radial elasticity coefficient of the radial resonant ring. For example, when the elastic bands have different elasticity coefficients, the elastic bands can be used individually. Alternatively, the elastic bands can be connected in series or in parallel, so that the radial resonant ring produces different radial elasticity coefficients.

In a preferred embodiment of the present invention, the measuring portion 12 is made of a non-elastic material, and is connected in series with the elastic band portion 13. Alternatively, the measuring portion 12 may be a combination of elastic material such as an elastic air bag 21 and non-elastic material, and then be connected in series with the elastic band portion 13. The pressure exerted by the tourniquet of the present invention on the pulse can be adjusted by the inflation and deflation of the elastic air bag 21. In this case, the tourniquet of the present invention can be connected with a pressure sensor of the measurement device 300 to sense the change of the internal air pressure of the tourniquet due to the sensed pulse beat. The elastic air bag 21 needs to be as thin as possible, and its elasticity coefficient also needs to help make the radial resonant ring generate better resonance with the harmonic of the blood pressure wave.

For example, the measuring portion 12 can be made of a non-elastic material, and is disposed overlapping the elastic air bag 21, that is, the elastic air bag 21 is disposed in the accomodating space 121, and the elastic air bag 21 is located on one side of the artery to be measured close to the organism relative to the measurement device 300. The elastic air bag 21 can not only overlap the measuring portion 12, but also extend to the whole or part of the elastic band portion 13. That is, the accomodating space 121 can extend from the measuring portion 12 to the elastic band portion 13, so that the tourniquet of the present invention can more accurately adjust the radial elasticity coefficient of the resultant radial resonant ring.

In another embodiment of the present invention, the elastic air bag 21 may not be disposed in the accomodating space 121, but only overlaps with the measuring portion 12 made of a non-elastic material, so that the elastic air bag 21 can be directly attached to the skin near the artery to be measured for measurement. The elastic air bag 21 can also extend to the whole or part of the elastic band portion 13.

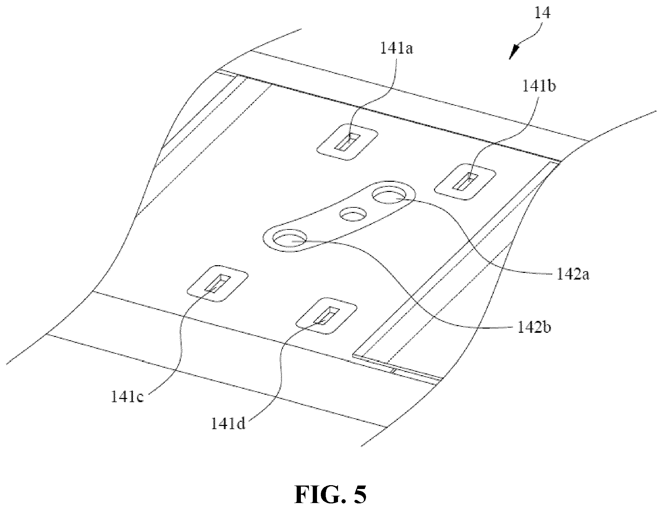

Referring to FIG. 5, in one embodiment of the tourniquet of the present invention, the covering bag 10 may include a fixing structure 14 for fixing and combining the measurement device 300 to the tourniquet of the present invention. In a preferred embodiment, the fixing structure 14 may be disposed on the measuring portion 12, and may include a plurality of fixing holes 141a, 141b, 141c, 141d for fixing the measurement device 300 and its elements. The elements of the measurement device 300 include a housing having a control circuit, a display screen, a battery, etc. In addition, the fixing structure may further include a plurality of holes 142a, 142b, which are used as the air inlet hole and the air outlet hole of the elastic air bag 21, or as the air passage holes of the measurement device such as a pressure sensor. The edges of the fixing holes 141a, 141b, 141c, 141d and the holes 142a, 142b are made of a non-slip material, such as leather, to increase the stability of fixation of the aforementioned elements.

In another preferred embodiment of the present invention, referring to FIG. 1, FIG. 3, and FIG. 5, the air bag device 20 may include a fixing element 22, and the fixing element can be coupled with the fixing structure to fix and combine the measurement device 300 to the tourniquet of the present invention, and at the same time to fix the air bag device 20 to the covering bag 10. This combination can increase the stability of fixation of the measurement device and the air bag device and the convenience of replacing the measurement device. The fixing element can form a plate structure and can be connected with the elastic air bag 21. The fixing element includes: an air inlet pipe 222a, an air outlet pipe 222b, and a plurality of fixation pillars 221a, 221b, 221c, 221d, wherein the plurality of fixation pillars 221a, 221b, 221c, 221d correspond to the fixing holes 141a, 141b, 141c, 141d for fixing the measurement device 300 and other elements of the measurement device 300. The air inlet pipe 222a and the air outlet pipe 222b correspond to the holes 142a and 142b respectively, and communicate with the elastic air bag 21. In addition, the plurality of fixation pillars 221a, 221b, 221c, 221d may be, but not limited to, a pillar with a wide end and a barb shape. After the air bag device 20 is put into the accomodating space 121, the air inlet pipe 222a, the air outlet pipe 222b, and the plurality of fixation pillars 221a, 221b, 221c, 221d respectively pass through corresponding holes 142a, 142b and fixing holes 141a, 141b, 141c, 141d, so that the tourniquet is coupled to the measurement device 300 by the plurality of fixation pillars 221a, 221b, 221c, 221d.

Furthermore, the surface of the measuring portion 12 contacting the elastic air bag 21 (e.g., the surface of the accomodating space 121) may have a non-slip material part, and may protrude outward along the fixing holes 141a, 141b, 141c, 141d and the edges of the holes 142a, 142b to increase the friction force between the elastic air bag 21 and the covering bag 10, thereby reducing the possibility of displacement between the measuring portion 12 and the elastic air bag 21.

In summary, the radial elasticity coefficient of the radial resonant ring formed by connecting the measuring portion 12 and the elastic band portion 13 in series must be matched with the frequency of each harmonic to be measured to generate resonance. Therefore, if the connection method causes the radial elasticity coefficient of the whole tourniquet of the present invention to deviate from the conditions required for measurement due to improper series connection or parallel connection, the accuracy for measuring and analyzing the blood pressure wave would be lost. Therefore, when making the tourniquet of the present invention, the elastic band portion 13 itself and the part of the elastic band portion 13 connecting the measuring portion 12 in series should be made by using an elastomeric knitting technique, such as jump knitting method or performing stitching with an elastic thread. For example, the measuring portion 12 and the elastic band portion 13 must be connected in series by using the elastomeric knitting technique, and the elastic band portion 13 cannot be penetrated to cause damage or stiffness and loss of elasticity.

In conclusion, the radial resonant ring formed by connecting the measuring portion and the elastic band portion of the tourniquet of the present disclosure in series can reduce the influence of non-radial vibration, so as to improve the sensitivity and accuracy of the radial resonance.

Although the present invention has been described with reference to the preferred embodiments, it will be apparent to those skilled in the art that a variety of modifications and changes in form and detail may be made. The scope of the present invention is defined by the appended claims.

## Claims

1. A tourniquet, comprising:
a covering bag (10), including a surrounding device, a measuring portion (12), and an elastic band portion (13) connected in series, wherein the measuring portion defines an accomodating space (121) and is made of a non-elastic material, and the elastic band portion is made of an elastic material; and
an air bag device (20), having an elastic air bag (20), the air bag device is put into the accomodating space such that the elastic air bag and the measuring portion overlap,
wherein the surrounding device includes a connecting member (111), so that the tourniquet forms a ring structure to locate the measuring portion at a pulse position to be measured,
**characterized in that**
the ring structure has a radial elasticity coefficient, and the radial elasticity coefficient is determined according to a frequency of a harmonic of a blood pressure wave to be measured.

2. The tourniquet according to claim 1, wherein the covering bag further includes a fixing structure, the air bag device includes a fixing element, and the fixing element is coupled with the fixing structure to fix the covering bag and the air bag device.

3. The tourniquet according to claim 2, wherein a surface of the accomodating space of the measuring portion has a non-slip material part, and the non-slip material part is in contact with the fixing element to prevent displacement of the air bag device.

4. The tourniquet according to claim 3, wherein the fixing structure of the covering bag has a plurality of holes and a plurality of fixing holes.

5. The tourniquet according to claim 4, wherein the fixing element of the air bag device includes an air inlet pipe, an air outlet pipe, and a plurality of fixation pillars, and the air inlet pipe and the air outlet pipe communicate with the elastic air bag.

6. The tourniquet according to claim 5, wherein after the air bag device is put into the accomodating space, the air inlet pipe, the air outlet pipe, and the plurality of fixation pillars respectively pass through corresponding holes and fixing holes, so that the tourniquet is coupled to a measurement device by the plurality of fixation pillars.

7. The tourniquet according to claim 1, wherein the measuring portion and the elastic band portion are connected in series by an elastomeric knitting technique.

8. The tourniquet according to claim 7, wherein the elastomeric knitting technique is a jump knitting method, or performs stitching with an elastic thread.

## Patentansprüche

1. Aderpresse, welche umfasst:
einen Umhüllungsbeutel (10), der eine Umschließungsvorrichtung, einen Messabschnitt (12) und einen elastischen Bandabschnitt (13) umfasst, die in Reihe miteinander verbunden sind, worin der Messabschnitt einen Aufnahmeraum (121) bildet und aus einem nicht elastischen Material besteht und der elastische Bandabschnitt aus einem elastischen Material besteht; und
eine Luftkisseneinrichtung (20) mit einem elastischen Luftkissen (20),
worin die Luftkisseneinrichtung so in den Aufnahmeraum eingesetzt ist, dass sich das elastische Luftkissen und der Messabschnitt überlappen,
worin die Umschließungsvorrichtung ein Verbindungselement (111) umfasst, sodass die Aderpresse eine Ringstruktur bildet, um den Messabschnitt an einer zu messenden Pulsposition anzuordnen,
**dadurch gekennzeichnet, dass**
die Ringstruktur einen radialen Elastizitätskoeffizienten aufweist und der radiale Elastizitätskoeffizient entsprechend einer Frequenz einer Harmonischen einer zu messenden Blutdruckwelle bestimmt wird.

2. Aderpresse nach Anspruch 1, worin der Umhüllungsbeutel ferner eine Befestigungsstruktur umfasst, die Luftkisseneinrichtung ein Befestigungselement umfasst und das Befestigungselement mit der Befestigungsstruktur gekoppelt ist, um den Umhüllungsbeutel und die Luftkisseneinrichtung zu befestigen.

3. Aderpresse gemäß Anspruch 2, worin eine Oberfläche des Aufnahmeraums des Messabschnitts einen Teil aus rutschfestem Material aufweist und der Teil aus rutschfestem Material mit dem Befestigungselement in Kontakt steht, um eine Verschiebung der Luftkisseneinrichtung zu verhindern.

4. Aderpresse gemäß Anspruch 3, worin die Befestigungsstruktur des Umhüllungsbeutels mehrere Löcher und mehrere Befestigungslöcher aufweist.

5. Aderpresse nach Anspruch 4, worin das Befestigungselement der Luftkisseneinrichtung ein Lufteinlassrohr, ein Luftauslassrohr und mehrere Befestigungssäulen umfasst und das Lufteinlassrohr sowie das Luftauslassrohr mit dem elastischen Luftkissen in Verbindung stehen.

6. Aderpresse nach Anspruch 5, worin nach dem Einsetzen der Luftkisseneinrichtung in den Aufnahmeraum das Lufteinlassrohr, das Luftauslassrohr und die mehreren Befestigungssäulen jeweils durch entsprechende Löcher und Befestigungslöcher verlaufen, sodass die Aderpresse über die mehreren Befestigungssäulen mit einer Messvorrichtung verbunden ist.

7. Aderpresse nach Anspruch 1, worin der Messabschnitt und der elastische Bandabschnitt durch ein Elastomer-Strickverfahren in Reihe verbunden sind.

8. Aderpresse nach Anspruch 7, worin das Elastomer-Strickverfahren ein Sprungstrickverfahren ist oder das Zusammennähen mit einem elastischen Faden umfasst.

## Revendications

1. Garrot, comprenant:
un sac de recouvrement (10), comprenant un dispositif d'enveloppement, une partie de mesure (12) et une partie formant bande élastique (13) reliées en série, dans lequel la partie de mesure définit un espace de logement (121) et est réalisée en un matériau non élastique, et la partie formant bande élastique est réalisée en un matériau élastique; et
un dispositif à coussin gonflable (20), comportant un coussin gonflable élastique (20),
le dispositif à coussin gonflable étant placé dans l'espace de logement de telle sorte que le coussin gonflable élastique et la partie de mesure se chevauchent,
dans lequel le dispositif d'enveloppement comprend un élément de liaison (111), de sorte que le garrot forme une structure annulaire permettant de positionner la partie de mesure au niveau d'un point de pouls à mesurer,
**caractérisé en ce que**
la structure annulaire présente un coefficient d'élasticité radiale, et ce coefficient d'élasticité radiale est déterminé en fonction d'une fréquence d'une harmonique d'une onde de pression artérielle à mesurer.

2. Garrot selon la revendication 1, dans lequel le sac de recouvrement comprend en outre une structure de fixation, le dispositif à coussin gonflable comprend un élément de fixation, et l'élément de fixation est couplé à la structure de fixation pour fixer le sac de recouvrement et le dispositif à coussin gonflable.

3. Garrot selon la revendication 2, dans lequel une surface de l'espace de logement de la partie de mesure comporte une partie en matériau antidérapant, et ladite partie en matériau antidérapant est en contact avec l'élément de fixation afin d'empêcher tout déplacement du dispositif à coussin gonflable.

4. Garrot selon la revendication 3, dans lequel la structure de fixation du sac de recouvrement comporte une pluralité de trous et une pluralité de trous de fixation.

5. Garrot selon la revendication 4, dans lequel l'élément de fixation du dispositif à coussin gonflable comprend un tuyau d'entrée d'air, un tuyau de sortie d'air et une pluralité de montants de fixation, et le tuyau d'entrée d'air et le tuyau de sortie d'air communiquent avec le coussin gonflable élastique.

6. Garrot selon la revendication 5, dans lequel, une fois le dispositif à coussin gonflable placé dans l'espace de logement, le tuyau d'admission d'air, le tuyau de sortie d'air et la pluralité de montants de fixation passent respectivement à travers les trous et les trous de fixation correspondants, de sorte que le garrot est couplé à un dispositif de mesure par la pluralité de montants de fixation.

7. Garrot selon la revendication 1, dans lequel la partie de mesure et la partie formant bande élastique sont reliées en série par une technique de tricotage élastomère.

8. Garrot selon la revendication 7, dans lequel la technique de tricotage élastomère est un procédé de tricotage par saut, ou consiste à réaliser des coutures avec un fil élastique.
